(19) Europäisches Patentamt
European Patent Office
Office européen des brevets

(11) Publication number: **0 435 617 A1**

(12) **EUROPEAN PATENT APPLICATION**

(21) Application number: **90314166.1**

(22) Date of filing: **21.12.90**

(51) Int. Cl.$^5$: **C12N 15/63**, C07K 13/00, C12N 15/86, C12N 5/10, C12N 1/21, C12N 15/12, C12P 21/08, A61K 39/00

(30) Priority: **21.12.89 US 454080**
**07.12.90 US 624124**

(43) Date of publication of application:
**03.07.91 Bulletin 91/27**

(84) Designated Contracting States:
**AT BE CH DE DK ES FR GB GR IT LI NL SE**

(71) Applicant: **THE UNIVERSITY OF GEORGIA RESEARCH FOUNDATION, INC.**
**612 Boyd Graduate Studies Research Center, DW Brooks Drive**
**Athens, Georgia 30602 (US)**

(72) Inventor: **Ivarie, Robert D.**
**54 Jackson Street**
**Watkinsville, Georgia 30677 (US)**
Inventor: **Morris, Julie A.**
**54 Jackson Street**
**Watkinsville, Georgia 30677 (US)**
Inventor: **Clark, Theodore G.**
**190 Morton Avenue**
**Athens, Georgia 30605 (US)**
Inventor: **Smith, Charles K.**
**5550 Arrowhead Dr.**
**Greenfield, Indiana 46140 (US)**
Inventor: **Heath, William F., Jr.**
**9502 San Jacinto Dr.**
**Indianapolis, Indiana 46250 (US)**

(74) Representative: **Fisher, Adrian John et al**
**CARPMAELS & RANSFORD 43 Bloomsbury Square**
**London WC1A 2RA (GB)**

(54) **Bovine myogenic factor gene.**

(57) The invention provides cloned DNA comprising a nucleotide sequence encoding bovine myogenic factor, which is implicated in the control of myogenesis. The invention also provides DNA comprising the promoter of the bovine myogenic factor gene.

EP 0 435 617 A1

Restriction map of bmyf gene and flanking sequences in recombinant clone λDIIBmyf1

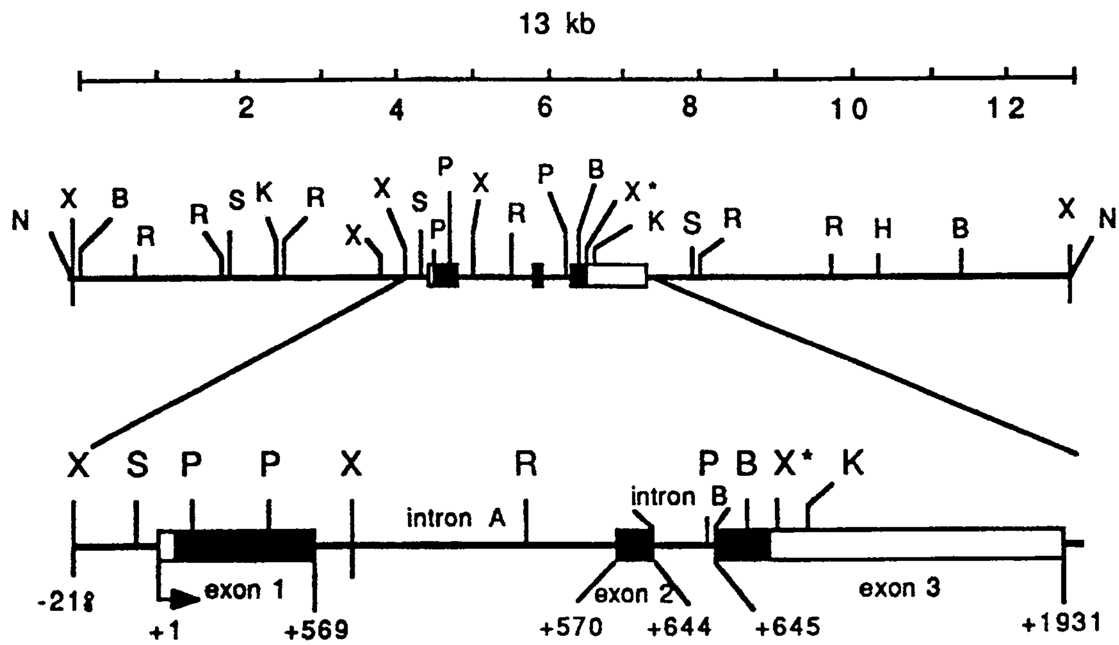


Positive (+) numbers identify nucleotide positions in bmyf cDNA sequence; minus (-) numbers identify nucleotide positions upstream from +1 of bmyf cDNA.

Restriction enzyme sites: B, BamHI
H, HindIII
K, KpnI
N, NotI
P, PstI
R, EcoRI
S, SacI
X, XbaI

amino acid coding

5' or 3' untranslated

intron A = ~1,000 bp
intron B = ~390 bp

FIGURE 1

# BOVINE MYOGENIC FACTOR GENE

## RELATEDNESS OF THE APPLICATION

The subject application is a continuation-in-part of a copending U.S. application filed December 7, 1990 (serial number unassigned), which is a continuation-in-part of copending U.S. Serial No. 454,080, filed December 21, 1989.

## BACKGROUND OF THE INVENTION

The present invention relates to the cloning, expression, expression products, regulation and genetic engineering of a gene involved in differentiation of precursor cells to muscle cells (myogenesis).

Myogenesis at the cellular level. Myogenesis can be broken into three general steps (see, Emerson, C. et al. (eds.) (1986) Molecular Biology of Muscle Development, Alan R. Liss, Inc., N.Y.). First, a mesodermal cell is converted to a proliferating myoblast. Second, myoblasts fuse to form multinucleated myotubes in which muscle-specific genes have been activated. In the first step, determination, cells and all their descendants are committed to the myogenic lineage. In the second, terminal differentiation, myoblasts form nondividing myotubes and activate the set of muscle-specific genes. A later third step involves maturation in which a complex series of regulatory events occur generating the various muscle protein isoforms. Proliferation myoblasts themselves appear to be heterogeneous in that subclones can be identified that are committed to activation of fast or slow twitch embryonic heavy chain myosin genes.

Steps leading to the origin of the mesodermal cell were not included in the pathway, but many of the details of the cellular pathway to skeletal muscle have been worked out in the early embryo. Skeletal muscle derives from the middle or mesodermal layer of the three germ cell layers (ecto-, endoand mesoderm), that give rise to the organism. Mesoderm is a pluripotent tissue that differentiates into a multitude of cell types besides skeletal muscle, including bone, cartilage and cardiac muscle ; parts of the eye, teeth, kidney, and gonads ; and the many cells of the hematopoietic pathway (erythrocytes, lymphocytes, macrophages, etc.). Mesoderm is also identified by where it occurs in various regions of the early embryo (dorsal, head, lateral) and it is dorsal mesoderm that produces morphologically visible somites and myotomes from which skeletal muscle differentiates. Determination, therefore, appears to involve conversion of dorsal mesodermal cells to proliferating myoblasts, thereby committing the mesodermal cell and its descendants to a myogenic lineage.

One commercial interest in genes controlling myogenesis includes their use for intervening to ameliorate muscle injury. Muscle contains satellite cells that are dormant myoblasts. New medicines might be developed that would enhance the rate of wound healing, especially of traumatic injury to muscle, via the ability to influence satellite cells to populate the site of injury and differentiate into myotubes. Another commercial interest centers on farm animals where myogenesis genes can be used to improve productivity with greater efficiency and reduced use of feed additives. Currently, such additives as estrogens and antibiotics are widely used to maximize muscling and weight gain. However, their use is frequently criticized because of potential residues in the human diet and for proliferation of antibiotic resistant microorganisms. Myogenesis genes can also be influenced pre- and post-natally in domestic farm animals to reduce fat deposition and increase muscling resulting in leaner meat (e.g., lower fat and cholesterol), and a much healthier human diet. There are also commercial diagnostic applications in veterinary and human medicine for many of the materials developed from myogenesis genes.

Recently, significant progress has been made toward isolating genes involved in establishing the muscle cell lineage taking advantage of an unusual property of mouse C3H10T1/2 cells in response to a potent inhibitor of DNA methylation (5-azacytidine). After treatment with the agent, colonies that contain multinucleated myotubes arise among the rare survivors (Constantides, P.G. et al. (1977) Nature 261 : 364 ; Taylor, S.M. and Jones, P.A. (1979) Cell 17 : 771 ; Jones, P.A. and Taylor, J.M. (1980) Cell 20 : 85). Unfused myoblasts can be isolated, stably propagated and induced to differentiate into myotubes in which muscle-specific genes have been activated, e.g., myosin heavy chain, myosin light chains (1emb, 1, 2, 3), α-actin, α, β-tropomyosin, and troponin C and T (Konieczny and Emerson (1981) Cell. 38 : 791-800).

These observations suggest a simple "master gene" hypothesis (see, Konieczny, S.F. et al. in Emerson et al., supra) based in part on the fact that genes that are undermethylated at cytosine residues are usually actively transcribed, while genes that are heavily methylated are transcriptionally inactive. According to the model, a single gene (i.e., a myogenic determination gene) is activated by demethylation and converts the 10T1/2 cell to a proliferating myoblast that can differentiate into myotubes. Confirmation of the model occurred when it was shown that DNA from myoblasts (quail and mouse) but not from nonmuscle cells could transform 10T1/2 cells to myoblasts when introduced by DNA-mediated transfection (Konieczny, S.F. et al. in Emerson et al. supra ; Lassar, A.B. et al. (1986) Cell 47 : 649-656). Unmethylated human DNA could also drive 10T1/2 cells down the myogenic lineage (Pinney, D.F. et al. (1988) Cell 53 : 781). Given that DNA from widely divergent species (birds to man) functioned identically in the transfection assays, there was remarkable conservation of genetic function.

The myoD gene family. Using DNA-mediated transfection of 10T1/2 cells as an assay, two different genes, termed myoD (Davis, R.L. et al. (1987) Cell 51 : 987) and myd (Pinney, D.F. et al., 1988), were initially identified which convert 10T1/2 cells into myoblasts that are capable of myotube formation. The cDNA to myoD1 was cloned from a subtracted cDNA library from 10T1/2 myoblasts and has been sequenced (Davis et al., 1987). Its myogenic activity was assayed as a chimeric gene using a retroviral promoter. Myd has not yet been cloned. By hybridization criteria on Southern blots, myoD and myd are not related to each other (Pinney et al., 1988). Biochemical studies have shown that myoD is a nuclear phosphoprotein that turns over with a half-life of 30-60 min (Tapscott, S.J. et al. (1988) Science 242 : 05). Its transcript is also unstable (Thayer, M.J. et al. (1989) Cell 58 : 21).

## BRIEF DESCRIPTION OF THE DRAWINGS

Figure 1 is a restriction map of the bmyf gene and flanking sequences in recombinant clone lambda D-11Bmyf1.

## SUMMARY OF THE INVENTION

A novel gene implicated in control of myogenesis, named herein for its expression product bovine myogenic factor (bmyf), has been isolated and characterized as cDNA.

The bmyf cDNA was unexpectedly isolated in a screen for sequences homologous to MyoD. The invention provides the bmyf coding sequence, bmyf cDNA, bmyf genomic DNA, the promoter of the bmyf gene, bmyf protein, polyclonal and monoclonal antibodies to bmyf protein, chimeric gene constructs of bmyf promoter with a heterologous gene to be regulated as bmyf is regulated in vivo, and chimeric gene constructs of an exogenous promoter and the bmyf coding region to express bmyf under conditions other than those which normally induce bmyf. The cDNA encoding bmyf is useful in various art-recognized expression systems for synthesis of bmyf protein in quantity. The cDNA is also useful in constructs to improve muscle maintenance or for genetic modifications to enhance weight gain and meat production efficiency. For example, transfected cell lines having the described chimeric gene constructs can be used for screening compounds for possible effects on myogenesis. The bmyf genomic gene is similarly useful, particularly for gene replacement and gene therapy uses. The bmyf promoter is particularly useful as a regulator of reporter genes that are desired to be activated or repressed in concert with other myogenesis genes. The promoter is also useful for assay systems to measure the effects of compounds as potential regulators of myogenesis. Screening for compounds affecting myogenesis, as therapeutic agents or regulators of muscle development, can be undertaken using constructs combining the bmyf promoter and a suitable reporter gene. These constructs can be placed in various cell lines by DNA transformation. Transformed cells are models for screening compounds affecting the activity of the bmyf protein or the bmyf promoter. The bmyf protein can itself serve as a pharmacologic agent, although its use as a model for development of smaller molecules having similar effects is more likely. The bmyf protein is also useful as an antigen for the production and selection of polyclonal and monoclonal antibodies to bmyf. The antibodies themselves are useful as a diagnostic tool to assay for bmyf in cells under normal and pathological states. The effect of various drug treatments on the bmyf promoter or protein can be monitored by means of antibodies to bmyf. Monoclonal antibodies directed to different bmyf epitopes are useful in mapping functional domains of the bmyf protein. Chimeric gene constructs of the bmyf promoter and a heterologous coding sequence provide a test system to measure effects of various compounds on bmyf expression in normal and pathologic states as well as in transfected cell lines. Chimeric gene constructs of an exogenous promoter and the bmyf coding sequence (either cDNA or genomic) are useful for therapeutic replacement in hereditary pathologic conditions characterized by abnormal expression, or to provide higher expression levels to enhance meat production, e.g., under control of an external stimulus such as a feed additive. The foregoing are illustrative but not exhaustive instances of the uses to be made of products of the invention.

Established and well-characterized techniques known in the art are available to those of ordinary skill to isolate and characterize the various products of the invention, based on availability and characterization of bmyf cDNA. For example bmyf cDNA can serve as a probe to isolate genomic bmyf DNA from bovine genomic libraries. Many expression systems are known in the art for expressing bmyf cDNA to produce bmyf protein, including fusion proteins to bacterial trpE gene protein or in the Baculovirus eukaryotic expression system. Isolating a bmyf genomic clone permits identification of the elements controlling bmyf gene transcription, including the promoter of the bmyf gene. After sequencing by well-known techniques, the promoter can be used in chimeric constructions combining the promoter with a heterologous coding sequence. The disclosed cDNA coding sequence also enables those of ordinary skill in the art to construct chimeric genes in which exogenous or heterologous promoters, such as a retrovirus or muscle-specifc gene promoter, controls expression of bmyf coding sequences. Known methods for making polyclonal or monoclonal antibodies are available, using the entire bmyf protein or various subregions provided by peptide synthesis as an antigen. Consequently the various products of the invention will be readily available to those of ordinary skill applied to the data, information and teachings herein disclosed. Conservative nucleotide sequence changes (those which do not result in altered amino acid sequence) can also be introduced into the DNA sequences using known techniques either to increase or decrease the activity of the bmyf promoter or polypeptide, or to increase or decrease the stability of the bmyf polypeptide or mRNA.

## DETAILED DESCRIPTION OF THE INVENTION

In general the terminology used herein is standard, as understood by those ordinarily skilled in the fields of molecular biology, genetics and cloning. For added clarity, certain terms are further defined. Standard abbreviations are used, such as approved by scientific journals in the field (Nature, Cell, Proceedings of the National Academy of Science USA, Science, etc.)

Myogenesis is the term applied to a multi-step process of cell differentiation whereby non-muscle stem cells are converted to muscle cells. Myogenesis is recognized by any of several criteria, such as myoblast formation, expression of muscle-specific genes into mRNA or proteins (myosin heavy and light chains, alpha actin, alpha and beta tropomyosin and troponin C and T), myotube formation, syncytium formation and activation of muscle-specific promoters. A currently convenient assay for an early step in myogenesis involves the publicly available embryonic mouse fibroblast cell line C3H10T1/2. C3H10T1/2 cells undergo myogenic conversion upon transfection with an expressible myogenesis gene such as MyoD or by 5-azacyticine treatment. In the present invention it is shown that transient transfection of C3H10T1/2 cells with bmyf cDNA in an expression vector such as pEMSV-bmyf also elicits a myogenic response in C3H10T1/2 cells.

Promoter is used herein to denote the region of a gene which functions in controlling transcription of the gene. A promoter is typically located near the 5′-end of the coding region (that region coding for the amino-terminal part of the encoded protein.) As is known in the art, the location of a promoter can be deduced from sequence analysis of genomic DNA and by other recognized techniques (S1 mapping, DNAseI footprinting, gel retardation assays and transient transfection with promoters and reporter genes). Promoters vary in size and often include sequences for more than one regulatory function, e.g., those involving tissue specificity, response to inducers, repressors and activators, etc.

A bmyf-active promoter is a nucleotide sequence variant of the bmyf promoter sequence, having at least 80% homology to the bmyf promoter sequence and having essentially the same function as the bmyf promoter. The bmyf promoter controls the transcription of the bmyf gene and includes the RNA Polymerase binding site and regulatory regions for activation and/or repression of transcription of the bmyf gene. Regulatory regions can be located 5′ to the structural gene, within the structural gene (including its introns), and/or 3′ to the structural gene. As discussed hereinbelow in the examples, regulatory regions of the promoter and their functions can be identified by methods known in the art.

Coding region is the term used herein to denote that part of a gene which encodes a protein. In cDNA a single polypeptide chain is encoded by a continuous, uninterrupted coding region. In genomic DNA (e.g., in the gene itself), the coding region is usually interrupted by one or more introns, which are non-coding intervening sequences present in genes but absent from the mRNA. A coding region is under control of a promoter when that promoter controls expression of the protein encoded by the coding region.

Genomic DNA is that cloned directly from the chromosomal DNA of an organism. The bmyf gene is contained in genomic DNA which has been cloned from the chromosomal DNA of the organism. As such it represents the structure of the gene in the chromosome of the organism, prior to transcription.

Expression means the detectable manifestation of the presence of a gene in a cell or organism. Expression has occurred whenever a phenotype attributable to the gene is observed. More directly, gene expression is the synthesis of a gene product, either by transcription into mRNA or by translation of the mRNA into a protein.

Chimeric gene is the term used herein to indicate a man-made gene constructed by combining functional gene components obtained from different sources. For example, a chimeric gene can be constructed by combining the bmyf promoter and the coding region of another gene. Such a coding region is termed a heterologous coding region because it is not regulated by the bmyf promoter in naturally-occurring cells. Where the heterologous coding region provides a readily detectable product when expressed, the chimeric gene is used as a reporter gene to measure the activity of the promoter under experimental conditions. Another example of a chimeric gene is one constructed by combining an exogenous, or heterologous promoter with the bmyf coding region. An exogenous promoter is one which does not regulate the bmyf gene in naturally-occurring cells.

The bmyf protein is the expression product of the bmyf gene. The deduced amino acid seqence of the bmyf protein is shown in SEQ ID NO. 3.

A bmyf-active protein is an amino acid sequence variant of bmyf protein having at least about 80% sequence identity with bmyf protein and having essentially the same activity. Thus, bmyf protein is known to function as a regulatory protein with a fundamental role in controlling myogenesis in mammalian cells. Further studies will establish that function more quantitatively and will elucidate a molecular mechanism for its activity. It will be understood that many amino acid sequence variants are possible having essentially the same activity. Such variants include, but are not limited to, conservative amino acid substitutions, as well as substitutions and deletions in non-critical regions of the protein. To the extent such variants possess essentially the same activity as bmyf protein itself (distinguishable from other proteins of myogenesis and quantitatively similar), they are termed bmyf-active proteins and are considered to be within the scope of the invention.

Methods used herein are either specifically referenced or sufficiently well known as to be available in one of several published collections of methods, for example, Maniatis et al. Molecular Cloning, a Laboratory Manual, Cold Spring Harbor Laboratory, Cold Spring Harbor, New York (1982) ; Genetic Engineering, Plenum Press, New York (1979) ; Weir, (ed.) (1986) Handboock of Experimental Immunology in Four Volumes, 4th Ed., Blackwell Scientific Publications, Oxford ; and the mutivolume Methods in Enzymology, published by Academic Press, New York.

Various cell lines and plasmid vectors have been disclosed. All are publicly available. The cells or tissues serving as source of bmyf cDNA or the bmyf gene are readily available. The plasmids, cloning vehicles and cells used for cloning transfer and expression were selected for operating convenience. Substitutes can be used at any step, bearing in mind the desired properties, according to criteria and properties known to those of ordinary skill in the relevant arts.

The following Examples detail the cloning and characterization of bmyf cDNA and other products of the invention.

Example 1 cDNA Library Construction and Screening.

Bovine myoblasts were isolated from fetal thigh muscle as described by Gospodarowicz et al. (1976) J. Cell. Biol. 70 : 395-405, except that selection with cytochalasin was omitted. Cells were lysed in 4 M guanidine thiocyanate, 0.5% (v/v) Sarkosyl, 0.025 M EDTA, and 0.1 M β-mercaptoethanol. RNA was isolated by sedimentation through CsCl as described (Chirgwin et al. (1979) Biochemistry 18 : 5294-5299 ; Freeman et al. (1983) Proc. Natl. Acad. Sci. USA 80 : 4094-4098. RNA pellets were dissolved in 10 mM Tris·HCl (pH 7.6), 1 mM EDTA, 5% (v/v) Sarkosyl and 5% (v/v) phenol, then extracted in phenol : chloroform : isoamyl alcohol (50: 49 : 1). Following ethanol precipitation, RNA was dissolved in deionized water and stored at -70°C. PolyA+ RNA was isolated by chromatography on oligo(dT)-cellulose and used to synthesize a first-strand cDNA with AMV reverse transcriptase and oligo(dT) as a primer (Berger and Kimmel (1987) Meth. Enzymol. 152) second-strand synthesis was carried out with DNA polymerase I in the presence of RNase H (Gubler (1987) Meth. Enzymol. 152 : 330-335). cDNAs averaging > 500 bp in length were isolated by chromatography on CL-4B sepharose, methylated and blunt-end ligated with EcoRI linkers. Linkered cDNA was then digested with EcoRI, fractionated by size and cloned into the EcoRI site of lambda gt10. Recombinant phage DNA was packaged using commercial extracts (Packagene™ system ; Promega, Madison, WI) and libraries were amplified prior to screening.

A phage library containing aproximately $3 \times 10^4$ recombinants was exhaustively screened with a$^{32}$ P-labelled 274 bp fragment of the mouse MyoD1 cDNA amplified via the polymerase chain reaction (PCR) using pVZC11b plasmid DNA as a template (Sakai et al. (1985) Science 230 : 1350-1354 ; Mullis and Faloona (1987) Meth. Enzymol. 155 : 335-350). A pair of degenerate 18 mer primers beginning at nt 339 and 612 of the sense and antisense cDNA strands, respectively, were synthesized and used to amplify the 274 bp fragment. The fragment, which spanned the cys/his, basic and myc homology regions of MyoD1, was labelled to high specific activity (> $10^9$ spm/μg) with $^{32}$P-(α)dATP using random oligonucleotide priming (Feinberg and Vogelstein (1983) Anal. Biochem. 132 : 6-13. For library screening, plaques were bound to nylon filters (Micron Separations, Inc.) using standard procedures (Carlock (1986) Focus 8 : 6-8). Filters were baked at 70°C for 60-90 min, and prehybridized at 50°C for 6-12 hr in 6X SSC (0.9 M NaCl, 0.09 M Na-citrate pH 7.0), 0.05% (w/v) SDS, 0.25% (w/v) Blotto, 1X Denhardt's solution (0.02% Ficoll (w/v), 0.02% polyvinylpyrrolidone (w/v), 0.02% (w/v) BSA), 0.05% (w/v) sodium pyrophosphate, and 5 mg/ml denatured salmon sperm DNA. Hybridization was carried out at 50°C for 24 hr with denatured probe at $10^6$ cpm/ml in buffer containing 6X SSC, 0.5% (w/v) SDS, and 0.05% (w/v) sodium pyrophosphate. Filters were then washed twice at room temperature in 2X SSC, 0.1% (w/v) SDS, once at 54°C in 1X SSC, 0.1% (w/v) SDS, and once at 54°C in 0.5X SSC, 0.1% (w/v) SDS. Each wash lasted 15-30 min. After exposing filters to kodak X-OMAT film, positive recombinants were purified by serial dilution and rescreened under conditions described above (Carlock et al., 1986).

Example 2 Characterization of cDNA Clones.

Recombinant phage DNA was digested with BamHI, electrophoresed on 0.7% agarose gels and transferred to Biotrans nylon filters (ICN Biomedicals Inc., Costa Mesa, CA) in 1 M ammonium acetate and 0.02 N NaoH (Rigaud et al. (1987) Nucl. Acids Res. 15 : 857). Filters were irradiated with UV, baked at 70°C and hybridized to the 274 bp probe essentially as described above for library screening. Recombinant inserts were subcloned into the EcoRI site of pGEMTM-3zf(-) (Promega) for restriction endonuclease mapping. Various subfragments were subcloned into M13 for sequencing both strands by the dideoxy method of Sanger et al. (1987). Where restriction sites were lacking, particularly in the in the 3'-untranslated region of bmyf, 17mers were synthesized to complete the sequencing.

The complete nucleotide sequence of the bmyf insert is shown in SEQ ID NO. 2. It contains 1,889 bases (exclusive of its short polyA-tail) and includes a long open reading frame of 765 nt beginning with a potential ATG start site at nt 69. The deduced amino acid sequence can encode a protein of 255 amino acids identical in size with the protein from the longest open reading frame of the human myf-5 cDNA (Braun et al. (1989) EMBO J. 8 : 701-709). Bmyf and human myf-5 share extensive homology particularly in their coding regions, with 96% identity at the amino acid and 92% identity at the nt sequence levels, respectively. Both cDNAs contain short stretches (68 nt for bmyf, and 42 nt for human myf-5) upstream of the potential ATG start codons in each. These regions are also highly related showing 82% sequence conservation. Although it is not known whether the first nt is the actual 5' transcriptional start site of the bmyf mRNA, an independently cloned and smaller bovine cDNA (clone 7) contains the identical 5' sequence including the first nt, suggesting that bmyf is indeed a full-length cDNA.

The principal difference between bmyf and human myf-5 occurs in the 3'-untranslated region where bmyf is several hundred nt longer. From the stop codon to the AATAAA polyadenylation signal at nt 1407 in human myf-5 and at nt 1415 in bmyf, the two cDNAs are highly related and share 81% sequence similarity. Human myf-5 terminates within a short distance of this polyadenylation signal, while bmyf extends an additional 475 nt terminating near a second polyadenylation signal at nt 1872. Presumably, the human myf-5 cDNA came from a transcript terminating at the upstream site, while bmyf came from a transcript terminating at the downstream site. As described below, RNA from fetal skeletal muscle contains a 1.5 kb bmyf transcript apparently arising from adenylation at the AATAAA site at nt 1415.

Example 3 Northern Hybridization Analysis.

Various tissues were excised from 20 cm bovine fetuses and frozen in liquid nitrogen. Skeletal muscle came from hind and forelimbs. For RNA preparation, frozen tissue was ground to a fine powder and lysed in 5 M guanidine thiocyanate, 10 mM EDTA, 50 mM Tris·HCl (pH 7.5) and 8% (v/v) β-mercaptoethanol. RNA was precipitated directly from the lysate with 4 M LiCl as described (Cathala et al. (1983) DNA 2 : 329-335). After solubilization and extraction with phenol, RNA was ethanol precipitated and stored either as LiCl pellets or as aqueous solutions at -70°C (Cathala et al., 1983). PolyA+ RNA was isolated as above, electrophoresed on 1.1% agaroseformaldehyde gels, and transferred onto Biotrans nylon membranes in 20X SSC. Filters were UV-irradiated, baked at 70°C for 60-90 min, and hybridized to $^{32}$p-labelled cDNAs as follows. Filters were prehybridized for 4 hr at 65°C in 0.5 M sodium phosphate (pH 7.2), 7% SDS, 1% BSA, and 1 mM EDTA, then hybridized with base-denatured probe (at > $10^6$ cpm/ml) for 18-24 hr under the same conditions (Mahmoudi and Lin (1989) Biotechniques 7 : 331-333). Filters were washed twice at 65°C in 40 mM sodium phosphate buffer (pH 7.2), 5% (w/v) BSA, 5% (w/v) SDS and 1 mM EDTA for 30 min each, followed by two washes at 65°C in 40 mM sodium phosphate (pH 7.2), 1 mM EDTA and 1% (w/v) SDS (Mahmoudi and Lin, 1989). Probes were gel purified before use (Dretzen et al. (1981) Anal. Biochgem. 112 : 295-298).

When polyA+ RNA from fetal skeletal muscle was analyzed on Northern blots using the full-length bmyf cDNA as a probe, three RNA species were detected migrating at 1.5, 2 and 3 kb. The 1.5 kb band was the most abundant, being present at 2-3 times the level of the higher molecular weight species. The presence of three distinct bands on Northern blots suggested that transcripts from the bovine myf gene are differentially processed. For example, the 1.5 kb RNA band might be equivalent to transcripts which generated the human myf-5 cDNA and lack the region downstream of the proximal polyadenylation signal in bmyf at nt 1415. To test this, Northern blots of polyA+ RNA from bovine skeletal muscle were probed with a 354 bp AvaII/EcoRI fragment from the distal end of bmyf, and 3' to the polyadenylation signal at nt 1415. The distal 354 bp fragment hybridized to the 2 and 3 kb bands, but virtually no hybridization was seen to the 1.5 kb RNA, indicating that this species lacks the same 3′ sequence missing in the human myf-5 cDNA.

To determine whether bmyf is expressed in a tissue-specific manner, polyA+ RNAs from several fetal bovine tissues were subjected to Northern analysis and probed with the full-length bmyf cDNA. Only RNA from skeletal muscle gave rise to signals. Based on comparisons with the signal generated from known amounts of bmyf RNA synthesized from a pGEMTM vector containing the bmyf, insert, the actual levels of bmyf transcripts present in skeletal muscle are low and estimated to be 0.001%-0.0001% of total message. Little or no signal was detected in Northern blots of RNA from mouse azamyoblast cell lines at the levels of stringency used above.

Example 4 Cell Cultures and DNA Transfection.

C3H10T1/2 (clone 8) from the American Type Culture Collection was grown in DMEM (Cellgro™, Mediatech, Washington, D.C.) supplemented with 10% heat-inactivated FCS and 100 µg/ml kanamycin. Stable azamyoblast cell lines were generated from C3H10T1/2 cells by treatment with 3 mM 5-azacytidine for 24 hr and serial subcloning (Konieczny and Emerson, 1984). On plating at low density, > 90% of colonies derived from 91a and 56a azamyoblast subclones form large numbers of multinucleated syncitia which stain positive with monoclonal antibodies against skeletal muscle myosin heavy chain (MF-20, see below).

DNA transfections were carried out using cDNAs inserted into the EcoRI site between the Moloney sarcoma virus LTR and SV40 polyadenylation signals of pEMSV-scribe (Harland and Weintraub (1985) J. Cell Biol. 101: 1094-1099 ; Davis et al., 1987). Full-length bmyf cDNA was inserted in both sense and anti-sense orientations. For transient transfections (Wright et al. (1989) Cell 56 : 607-617), C3H10T1/2 cells were plated at $1 \times 10^5$ cells per 10 cm dish and grown for 2 days in DMEM containing 10% heat-inactivated fetal calf serum. Medium was changed 3 hr prior to transfection and plasmid DNA was added as calcium phosphate precipitates (7.5 µg/dish) as described (Gorman (1985) in DNA Cloning Vol II, IRL Press, Oxford UK, pp. 143-190). Cells were incubated 24 hr, washed with phosphate-buffered saline (137 mM NaCl, 2.7 mM KCl, 4.3 mM $Na_2HPO_4$, and 1.4 mM $KH_2PO_4$) and either maintained in differentiation medium (DMEM with 2% (v/v) heat-inactivated horse serum) or grown for two days in normal medium before incubation in differentiation medium. After 3 days, cells were fixed in 70% (v/v) ethanol, 3.7% (v/v) formaldehyde and 5% (v/v) glacial acetic acid, and reacted sequentially with monoclonal antibodies against the heavy chain of skeletal muscle myosin (MF-20, Bader et al., 1982) followed by goat anti-mouse IgG conjugated to alkaline phosphatase. Immunostaining was carried out with NBT and BCIP color reagents (Promega) according to the supplier's directions. Photomicrographs were taken with a Zeiss inverted microscope using Kodak Panatomic-X film.

Transient assays with the same vector have shown that the bovine myf cDNA can convert C3H10T1/2 cells into myoblasts following transfection. C3H10T1/2 cells were transfected with the expression vector described above carrying bmyf in its sense orientation, induced to differentiate for 3 days, then fixed and reacted with a monoclonal antibody against skeletal muscle myosin heavy chain. Cells transfected with the same vector carrying either bmyf in its anti-sense orientation, or mouse MyoD1 in its sense orientation served as negative and positive controls, respectively. A number of cells which reacted positively with the myosin antibody probe following transfection with the sense-oriented bmyf expression plasmid. No antibody-positive cells were observed in cultures transfected with the bovine myf expression plasmid in the anti-sense orientation. Cells converted to the muscle myosin phenotype with the bovine myf expression vector were present in two experiments at $10^{-3}$ and $10^{-4}$ frequency, which is several orders of magnitude higher than the spontaneous activation level in 10T1/2 cells (Lassar et al., 1986). Both mono- and multi-nucleate cells were seen that stained positive for heavy chain myosin.

Example 5 The structure of the bmyf gene and its proximal promoter.

Introns were deduced to be present in the bmyf gene by comparison of restriction maps of bmyf cDNA and of bovine fetal skeletal genomic DNA (Figure 1). To determine the precise location of introns within the bmyf gene, a nested set of sense and antisense bmyf cDNA primers was used to amplify bmyf genomic fragments via polymerase chain reaction, PCR (U.S. Patent No. 4,683,202). One amplified genomic fragment containing a larger intron (intron A in Figure 1) was subcloned and the precise site of the intron determined by partial sequencing by the dideoxy chain termination method. The fragment was deduced to contain an intron of about 1 kb between cDNA residues 569 and 570. Restriction mapping of genomic DNA (Figure 1) revealed EcoRI and XbaI sites residing in intron A. A second amplified fragment was found to contain a smaller intron (intron B of Figure 1) of about 300 nts.

The overall structure of the bmyf gene is quite similar to the herculin gene (J.H. Miner & B. Wold, PNAS USA 87 : 1089-1093 (1990)) which is contiguous to myf-5 in the mouse ; this similarity is consistent with the idea that the bmyf and herculin genes arose via an ancient gene duplication of an ancestral gene followed by sequence divergence.

To isolate the proximal bmyf promoter, "reverse" PCR was used to augment the region 5′ to the cDNA. Initially, fetal bovine skeletal muscle genomic DNA was digested with XbaI and EcoRI. As indicated above, XbaI and EcoRI restriction sites were determined to exist in intron A. The digest was then reannealed under conditions favoring ligation of circular molecules, so as to form a 930 bp circular DNA molecule consisting of the XbaI fragment extending from -218 relative to cDNA to +145 of intron A. Two primers, beginning at +466 sense and +218 antisense, were then used to augment via PCR the region between the two primers containing the XbaI site. This region included the region 5′ to the cDNA.

A 281 bp XbaI/PstI fragment derived from the 930 bp fragment was subcloned and sequenced by the dideoxy chain termination method. Its sequence revealed that the fragment contained nt 1-67 of the bmyf cDNA, thereby demonstrating that the part of the 930 bp fragment up to XbaI site at -218 was in fact 5′ to the cDNA and represented the proximal promoter.

Example 5.1 Isolation of the bmyf gene from bovine genomic DNA.

To isolate a fragment containing a genomic bmyf DNA insert, a commercially available bovine genomic library was screened by : (1) amplifying fragments containing the bmyf gene by PCR using intron A primers ; and (2) screening the amplified fragments of (1) with full length bmyf cDNA probe.

20,000 recombinants of the Stratagene 1 DASH™ II genomic library, comprising a partial Sau3A digest of bovine genomic DNA, were plated onto each of 10 large plates. The plate lysates were assayed for the presence of the bmyf gene by amplification by PCR using intron A primers. Four plates scored positive and one was exhaustively screened using a full length bmyf cDNA probe.

One of the positive plaques, lambda DIIbmyf1, contains an insert of about 13 kb whose partial restriction map (Figure 1) indicates that it harbors the bmyf gene. The bmyf gene (approximately 3 kb) is flanked on the 5′ side by a fragment of about 4 kb and on the 3′ side by a fragment of about 7 kb. The 13 kb clone has been confirmed as containing the bmyf gene by virtue of the fact that the 217 nts of the first exon demonstrate complete sequence homology with a sequence of the bmyf cDNA.

To sequence the bmyf coding and promoter regions, various regions of the 13 kb fragment were subcloned into the EcoRI polylinker sites of pGEM™-3zf and —zf vectors (Promega). Single stranded DNA was isolated and sequenced using the dideoxy chain termination method and 20 mer oligonucleotides to prime the synthesis reactions. A composite of the cDNA and available genomic sequences is sequentially (5′ to 3′) presented in SEQ ID NOS. 4-12. The cDNA nucleotide sequence and matching bmyf amino acid sequence appears in SEQ ID NO. 1. The cDNA nucleotide sequence appears in SEQ ID NO. 2. The bmyf amino acid sequence appears in SEQ ID NO. 3. In SEQ ID NOS. 1 and 2, the coding region of the cDNA sequence is indicated in codon triplets; the noncoding region of the cDNA is indicated in nucleotides arranged in blocks of 10.

SEQ ID NO. 4 begins near (but not not include) the BamHI site approximately -4500 nt upstream from position +1 of bmyf cDNA. SEQUENCE ID NO. 4 is 385 nucleotides in length.

SEQ ID NO. 5 begins at the SacI site (GAGCTC) approximately -2300 nt upstream from position +1 of bmyf cDNA. SEQ ID NO. 5 is 815 nucleotides long. Between SEQ ID NO. 5 and NO. 6 is an unsequenced segment of about 440 bp.

SEQ ID NO. 6 is the proximal promoter sequence beginning at -946 nt upstream from position +1 of bmyf cDNA ; i.e., this sequence patches directly onto position +1 of bmyf cDNA. Sequence information for the promoter region indicates that the promoter is GC-rich and contains three TATA motifs (at approximately -158, -114, and -60 upstream of the +1 of cDNA) that may serve as sites for binding transcription factor, TFIID, which is required for RNA Polymerase II-mediated transcription. Footprinting of the 5′ region to determine the TATA box likely used as the transcription start site is described hereinbelow.

SEQ ID NO. 7 is the section of bmyf cDNA beginning at position +1 and continuing to +569 of cDNA, the point at which intron A begins in the genomic DNA. The coding region of the cDNA sequence is indicated in codon triplets. The noncoding region of the cDNA is indicated nucleotides arranged in blocks of 10.

SEQ ID NO. 8 is the sequence encoding a portion of intron A. Intron A is about 1000 bp and has not been completely sequence. SEQ ID NO. 8 is 361 bp of the 5′ end of intron A. Following SEQ ID NO. 8 is an unsequenced region of about 400 bp.

SEQ ID NO. 9 is 258 bp of intron A downstream of the unsequenced 400 bp region.

SEQ ID NO. 10, which follows directly from SEQ ID NO. 9, begins at the coding region corresponding to postion +570 of bmyf cDNA.

SEQ ID NO. 11 is the sequence for all of intron B. Intron B is located in the genomic DNA at the point between bmyf cDNA positions +644 and +655. Intron B is 393 bp in length.

SEQ ID NO. 12 is the sequence for exon 3. Exon 3 begins at +66 of the cDNA. It is 1245 bp in length and extends thru the site of polyA addition.

Example 5.2 Mapping the transcription start site of the bmyf gene.

As described hereinabove, the proximal promoter region contains three TATA elements from which transcription may be initiated by RNA Polymerase II. RNAse protection experiments were conducted to identify the most likely start site.

An antisense RNA probe labelled with $^{32}P$ extending from the PstI site at +64 of the cDNA to the upstream XbaI site at -218 was annealed to total RNA from bovine fetal skeletal muscle (18 cm fetus). The annealed mixture was digested with RNAseA and T1. Protected, double stranded fragments were then denatured and resolved on sequencing gels and their size measured by comparison to a set of dideoxy sequencing reactions throughout the same region. By this method, it was determined that the transcription start site is about -185 nt upstream of the PstI site at +6 in the cDNA, or at the TATA motif at about -114 nt upstream of the cDNA +1.

Usage of the promoter may be higher in earlier fetuses than in older ones (e.g., 18-42 cm fetuses) as judged by probe signal intensities.

Example 6 Chimeric constructs.

Subclones of the bmyf gene that include only the bmyf coding region, or any other desired region of the bmyf gene can be constructed. For example, sequence information provided herein identifies appropriate restriction sites which can be used to construct and clone the desired segment. Other isolation techniques, not requiring sequence information of the desired fragment, are available, in particular the PCR, described in U.S. Patent No. 4,683,202. Synthetic primers having sequences bracketing a desired segment can be constructed, allowing the polymerase chain reaction to amplify virtually any specific desired segment of the bmyf gene, for example portions of the bmyf promoter. Furthermore, as is known in the art, the primer sequences can be so constructed as to introduce restriction sites of desired specificity adjacent to the amplified segment.

Chimeric gene constructs of segments of the bmyf promoter combined with segments of a heterologous gene, or constructs of exogenous promoters combined with the bmyf coding region can be created, as exemplified hereinbelow.

Further, other portions of the bmyf gene can be similarly subcloned and combined into chimeric functional genes. In this way, specific functional domains of the bmyf gene can be either added to other gene constructs or deleted from the bmyf gene to yield altered function, either by adding or removing the function associated with the subcloned domain.

Example 6.1 Chimeric bmyf promoter/CAT gene constructs.

A functional chimeric gene combining the bmyf promoter and a heterologous gene provides for regulation of transcription of the heterologus gene in the manner bmyf itself is regulated. If the heterologous coding gene is a reporter gene, e.g., encoding chloramphenicol acetyl transferase (CAT), the resulting construct serves to provide an easily measurable indication of stimuli and chemical compounds that affect bmyf expression, either by stimulating or repressing it.

To map functional regions of the bmyf promoter roughly, two overlapping bmyf promoter regions of different length are ligated to the chloramphenicol acetyltransferase (CAT) structural gene. These constructs are then transfected into cell lines and cells are assayed for CAT activity using $^{14}C$-chloramphenicol.

One construct contains the full-length 5′ flanking region of the 13 kb fragment, and extends from the unique BamHI site at -4.2 kb to the unique PstI site at +64. To construct the 4.2 kb promoter/CAT gene, the 4.2 kb fragment bears a bluntend at the 5′ BamHI site and a 3′ PstI site. pCAT™ is bluntended at the HindIII site in the polylinker and then cleaved with PstI to generate a site compatible for directional cloning of the 4.2 kb promoter fragment.

The second construct contains a proximal promoter segment extending from the -218 XbaI site to the +6 PstI site. The proximal promoter/CAT construct is created by ligating the 282 bp XbaI/PstI fragment to the pCAT polylinker site between the XbaI and PstI sites.

These constructs can each then be used to transiently transfect 10T1/2 cells using an expression vector such as pEMSV, as described hereinabove in Example 4, to determine if the fragment extending from the -4.2 kb BamHI site to the -218 XbaI site contains a functional region of the promoter.

Example 6.2 Chimeric bmyf promoter/CAT gene constructs useful in identification of bmyf promoter cis segments.

To identify regulatory regions of the bmyf promoter, the construct identified in Example 6.1 as containing a functional promoter region, and a series of deletion mutations of the construct, are transfected into cell lines and expressed. The series of deletion mutations consist of a progression of deletions of the 5′ end of the promoter towards the transcription start site.

Initially, the full length promoter/CAT gene construct is transiently transfected into a number of cell lines to determine which lines are capable of expressing the CAT gene off the bmyf promoter. For example, since the myf-5 gene is expressed in L6 and C2C12 cells (Wright, W. et al. (1989) Cell 56 : 607-671 ; Braun, T. et al. (EMBO J 8 : 3617-3615) ; peterson, C. et al. (1990) Cell 62 : 493-502), they are the most likely target cell lines for transfections with the bmyf promoter/CAT constructs. Further, even though a particular cell line may not express the CAT gene off the bmyf promoter, deletion studies with such a cell line may reveal those regions of the bmyf promoter which act as silencers, i.e., regions which activate the gene only by their removal.

The full-length bmyf promoter/CAT construct and its deletion derivatives are then transiently cotransfected with RSVlacZ (sambrook, J et al. (1989) Molecular Cloning : A Laboratory Manual (2nd ed.) ; and Webster, J. et al. (1989) EMBO J 8 : 1441-46) into the selected cell lines, and then assayed for CAT activity using $^{14}$C-chloramphenicol. β-galactosidase activity provides an internal control for transfection efficiency between lines (Webster, J. et al. (1989) EMBO J 8 : 1441-46). Transfection is by the calcium phosphate method (Clark, T. et al. (1990) Nucleic Acids Res. 18 : 3147-3153 ; Glover, D.M. (1985) DNA Cloning : A Practical Approach II, 143-190).

Once the key regions are located by deletion analysis, the cis, elements can be confirmed. The promoter regions believed to be necessary for promoter activity can be confirmed by gel shift in vitro assays using extracts from cell lines kown to express the bmyf Promoter/CAT construct.

Promoter fragments determined to contain cis elements are then subjected to DNAse footprinting to define the protected region precisely. DNAse footprinting is carried out by preparing two batches of the promoter fragment in question, one batch with and one without cell extract containing trans-acting regulatory elements ; the fragments in each batch have radiolabeled 3′ or 5′ ends ; the batches are briefly exposed to DNAse I to produce 1 cut per fragment on average ; each batch then undergoes gel resolution to identify those areas in which DNAse I cuts did not occur (protected by regulatory protein).

Example 6.3 Chimeric bmyf promoter/CAT constructs useful in identification of enhancer regions.

The constructs of Examples 6.1 and 6.2 can be further modified to include fragments believed to contain bmyf enhancer sequences. These sequences can be located in 5′ and 3′ flanking regions, as well as in introns. Enhancers typically exhibit cell type specificity. The pCAT vectors contain several unique restriction sites downstream of the CAT gene where such enhancer sequences can be inserted. These restriction sites include EcoRI and BamHI. The resulting construct can be transiently transfected into 10T1/2 cells and other cell lines by methods described herein or known in the art, and bmyf protein production assayed, to determine the cell-type specificity for the enhancer fragment at issue.

Example 6.4 Chimeric bmyf promoter/β-galactosidase gene constructs.

An alternate reporter heterologous gene is the β-galactosidase gene. Bmyf Promoter/β-gal gene constructs can be created by excising from pCH11O (Hall, C. et al. (1983) J. Mol. Appl. Genetics 2 : 101-109 ; and available from Pharmacia LKB) the lacZ gene on a BamHI/HindIII fragment, and directionally inserting the lacZ fragment into BamHI/HindIII-cleaved pSP72 (Promega). The 4.2 kb BamHI/PstI bmyf promoter fragment can then be inserted into pSP72 at XhoI and PvuII sites by blunt end ligation. Further, there are 7 restriction sites in pSP72 downstream of the site at which the lacZ fragment is inserted at which bmyf fragments (other than the 4.2 kb 5′ fragment), comprising muscle-specific enhancers, can be inserted. The 7 restriction sites include SmaI, KpnI, SacI, EcoRI, ClaI, EcoRV and BglII. The β-galactosidase gene is useful as a reporter gene because of the color reactions associated with β-gal activity, and because antibody directed against β-gal is commercially available.

Example 6.5 Chimeric pMMTV-bmyf gene and pMMTV-myoD gene constructs.

A chimeric gene having an exogenous promoter combined with the bmyf coding region expresses bmyf under conditions that normally result in activity of that promoter. A chimeric gene of this sort makes it possible to express sufficient quantities of bmyf protein for isolation and further characterization.

For example, EcoRI restriction fragments containing the complete bmyf cDNA and the myoD coding regions were subcloned in pΩ5 vector (Morganstern, J. & Land, H. (1990) Nucleic Acids Res. 18 : 1068) harboring an enhancerless murine mammary tumor virus (MMTV) promoter which is inducible 20-to 50-fold by dexamethasone in mammalian cultured cells. Bmyf and myoD coding regions were inserted in the sense (pΩbmyf+ and pΩmyoD+) and antisense (pΩbmyf1⁻ and pΩmyoD⁻) orientations.

Example 7 Expression of bmyf in cell lines and purification.

The bmyf protein is obtained using any one of the known expression systems, in combination with a chimeric gene of the invention. For example, the coding region of either the bmyf gene or bmyf cDNA is combined with the trpE promoter of E. coli to obtain high level expression of bmyf protein in a bacterial culture system. Alternatively, the baculovirus expression system, using the known, highly active promoter of the viral coat protein gene, is used to produce desired quantities of bmyf protein in cultured insect cells.

Purification of the bmyf protein is accomplished by combining techniques known in the art. In highly active expression systems, the desired protein is the major expression product. The protein is identified by its size on electrophoresis gels. The protein is also identified and assayed by antibodies. Such antibodies are prepared, for example, by immunization with the protein in the gel band. Alternative subregions of 40-80 amino acids in length are synthesized using standard techniques known in the art. Specific antibodies are alternatively obtained by immunizing an appropriate host (rabbit of mouse) with an extract of the expression cells or synthesized peptides, then clearing the antibody preparation of non-bmyf antibodies by adsorption against control extracts of expression cells lacking bmyf, protein. The bmyf protein is purified by affinity adsorption, using antibodies bound to a solid phase support material. Other techniques of protein purification known in the art are employed as needed, to achieve desired purity. Purified bmyf protein is then characterized by standard techniques.

Example 7.1 Stable cell lines transfected with pMMTV-bmyf and pMMTV-myoD constructs.

The pMMTV-bmyf and pMMTV-myoD constructs described in Example 6.4 were used to cotransfect cell lines C3H10T$\frac{1}{2}$ and NIH3T3 (available from the American Type Culture Collection) with RSVneo (Gorman, C. (1985) in DNA Cloning : A Practical Approach II (ed. Glover, D.) pp. 143-190) to isolate stable lines that express bmyf and myoD in a steroid-inducible fashion. Transfection was carried out by the calcium phosphate method (Clark, T. et al. Nucleic Acids Res. (1990) 18 : 3147-3153 ; Glover, D.M., DNA Cloning : A practical approach II (1985)143-190). These lines have been determined to be dexamethasone-inducible for myoblast formation and for expression of the bmyf transcripts as assayed by Northern blots. These cell lines are used to analyze the turnover rates of the bmyf transcripts. Further, the bmyf protein produced and purified from these cell lines is used for further characterization of the protein, e.g., analyzing the phophorylation state and nuclear localization of the bmyf protein.

Example 8 Antibodies.

Both polyclonal and monoclonal antibodies to bmyf protein are obtained, using bmyf protein as an antigen. The methods and techniques employed are those routinely used in the art for immunization, antibody selection, purification, hybridoma screening and immunoassays. By choosing a combination of such methods as those ordinarily skilled in the art would apply, given the structural and functional characteristics of bmyf protein as established in the previous examples, antibodies suitable for such known techniques as immunoassay, staining, inactivation, affinity purification, epitope mapping and the like are produced and purified. A typical antibody preparation is a proteinaceous composition which contains either polyclonal or monoclonal antibodies, depending on the process used to obtain the antibody. An antibody specific for bmyf protein is one which binds to bmyf as is understood in the field of immunology. Since the bmyf protein shares regions of homology with other proteins, some antibodies specific for bmyf Protein may cross-react with other proteins to some extent. For some uses, cross-reactivity is tolerable, while for other uses, antibodies which do not cross-react measurably with other proteins are preferred.

Applicant has generated polyclonal rabbit antibodies to three synthesized peptides comprising amino acids 1-19, 101-118 and 154-169 (relative to the N-terminus). Regions likely to be antigenic were initially selected by using regions having an antigenic index of at least 1.000 as provided by the PEPTIDESTRUCTURE™ program (Jameson, B.A. & Wolf, H. (1988) Comput. Appl. Biosci. 4 : 181-186 ; Pearson, W.R. & Lippman, D. J. (1988) PNAS USA 85 : 2444-2448 ; available from University of Wisconsin, Madison). The antigenic index is a summation of peptide parameter information including hydrophilicity, surface accessibility, type of structure (e.g., α, β or β sheet) and Chou-Fasman character. Further selection of antigenic peptides for use in raising antibodies was done on the bases of (1) length of the antigenic region, (2) presence of strong β turns in predicted surface regions, (3) absence of cysteines within the antigenic region, and (4) lack of known homology with other proteins.

It has been found that antigenic regions of at least 15 residues are preferred because they are more likely to allow the presentation of the antigenic region in the conformation assumed in vivo. Also, a longer fragment potentially provides more epitopes for recognition by antibodies, thereby increasing the likelihood of obtaining specific antibodies. The presence of strong β turns in predicted surface regions is preferred because it has been found that strong β turns frequently retain their in vivo conformation in vitro. The absence of cysteines from the antigenic region is preferred so as to avoid reaction of the thiol groups with reagents used to secure antigenic peptides to particles. The presence of cysteines in regions flanking the antigenic region can be useful in securing the fragment to the particle or support. If cysteines are not present naturally in flanking regions, they can be inserted. It is preferred that the antigenic regions be non-homologous to other proteins so as to avoid cross-reactivity of antibodies with such other proteins. The TFASTA program (Pearson, W.R. & Lippman, D.J. (1988) PNAS USA85 : 2444-2448 ; available from the Univeristy of Wisconsin, Madison, Wisconsin) was used to compare the sequences of the antigenic regions with all amino acid sequences that could be translated from the nucleotide sequences of the EMBL/Genbank database. To avoid cross-reactivity with other proteins, the antigenic regions to be used for raising polyclonal antibodies were selected on the basis of lack of homology with any potential amino acid sequences translatable from Genbank nucleotide sequences. At the time such analysis was conducted, only murine myoD (and not bovine myoD) was available on Genbank.

For each of the selected peptides, antisera was raised by the following method : (1) injection i.m. in rabbits of 50 μg of the peptide in Freund's complete adjuvant ; (2) two weeks later, injection i.m. of 100 μg peptide in Freund's incomplete adjuvant ; (3) injection i.m. of 100 μg peptide in Freund's incomplete adjuvant weekly for three weeks ; (4) resting for three weeks ; (5) boosting with 100 μg peptide in Freund's incomplete adjuvant ; (6) resting for three weeks ; and (7) boosting with 100 μg peptide in Freund's incomplete adjuvant. Serum was then collected, and reactivity with bmyf and bovine myoD determined by immunoprecipitation.

For each of the antigenic peptides comprising amino acids 1-19, 101-118 and 154-169, the respective antisera were found to react with bmyf protein, and cross-react with myoD protein. The polyclonal antibody preparations can be further purified so as to reduce or eliminate those antibodies that cross-react with myoD. This can be accomplished by methods known in the art, such as antibody clearance using affinity chromatography methods employing myoD fixed to a substrate. For example, the antibody preparation can be passed through a chromatography column in which mammalian myoD protein or related proteins, or antigenic fragments thereof (including synthetic N-terminal fragments), are fixed to a solid support or substrate, resulting in the adherence of antibodies reactive to myoD to the column. Antibodies cross-reactive to other myogenic proteins can be removed in the same fashion, if necessary. The resulting eluate contains a greater proportion of antibodies specific to bmyf, protein and not reactive with bovine myoD.

SEQUENCE LISTING

(i) Applicant: University of Georgia Research Foundation

(ii) Title of Invention: Bovine Myogenic Factor Gene

(iii) Number of sequences: 12

(iv) Correspondence address:

(A) Addressee:Greenlee and Associates
(B) Street:5370 Manhattan Cir. Ste. 201
(C) City: Boulder
(D) State:Colorado
(E) Country:USA
(F) Zip: 80303

(v) Computer Readable Form:

(A) Medium type:
(B) Computer:
(C) Operating System:
(D) Software:

(vi) Current Application Data:

(A) Application number: unassigned
(B) Filing date:
(C) Classification: unassigned

(vii) Prior Application data:

(A) Application number: 454,080
(B) Filing date: December 21, 1989

(ix) Information for SEQ ID NO: 1

(i) Sequence characteristics:

(A) Length: nucleotide is 1931;
amino acid is 255

(B) Type: nucleotide and amino acid

(C) Strandedness: nucleotide is double stranded

(D) Topology: linear

```
CCAGGCTCCG GTTTCTCCCC TATCTATCTC TCTGCTGTCC AGGCCCACCC CTTGCCTCTC           60

TGCAGACG ATG GAC ATG ATG GAC GGC TGC CAG TTC TCG CCC TCT GAG TAC TTC TAC GAC GGC  122
         Met Asp Met Met Asp Gly Cys Gln Phe Ser Pro Ser Glu Tyr Phe Tyr Asp Gly
                         5                  10                  15

TCC TGC ATC CCA TCC CCC GAC GGT GAG TTC GGG GAC GAG TTT GAG CCG CGA GTG GCT GCT  182
Ser Cys Ile Pro Ser Pro Asp Gly Glu Phe Gly Asp Glu Phe Glu Pro Arg Val Ala Ala
    20                  25                  30                  35

TTC GGG GCT CAC AAG GCA GAC CTG CAA GGC TCA GAC GAG GAC GAG CAC GTG CGA GCA CCC  242
Phe Gly Ala His Lys Ala Asp Leu Gln Gly Ser Asp Glu Asp Glu His Val Arg Ala Pro
    40                  45                  50                  55

ACG GGC CAC CAC CAG GCC GGC CAC TGC CTC ATG TGG GCC TGC AAA GCA TGC AAA AGG AAG  302
Thr Gly His His Gln Ala Gly His Cys Leu Met Trp Ala Cys Lys Ala Cys Lys Arg Lys
    60                  65                  70                  75

TCC ACC ACC ATG GAT CGG CGG AAG GCG GCC ACC ATG CGC GAG CGG AGA CGC CTG AAG AAG  362
Ser Thr Thr Met Asp Arg Arg Lys Ala Ala Thr Met Arg Glu Arg Arg Arg Leu Lys Lys
    80                  85                  90                  95

GTC AAC CAG GCT TTC GAC ACG CTC AAG CGG TGC ACC ACG ACC AAC CCT AAC CAG AGG CTG  422
Val Asn Gln Ala Phe Asp Thr Leu Lys Arg Cys Thr Thr Thr Asn Pro Asn Gln Arg Leu
    100                 105                 110                 115

CCC AAG GTG GAG ATC CTC AGG AAT GCC ATC CGC TAC ATT GAG AGT CTG CAG GAG CTG CTT  482
Pro Lys Val Glu Ile Leu Arg Asn Ala Ile Arg Tyr Ile Glu Ser Leu Gln Glu Leu Leu
    120                 125                 130                 135

AGG GAA CAG GTG GAA AAC TAC TAT AGC CTG CCG GGG CAG AGC TGC TCT GAG CCC ACC AGC  542
Arg Glu Gln Val Glu Asn Tyr Tyr Ser Leu Pro Gly Gln Ser Cys Ser Glu Pro Thr Ser
    140                 145                 150                 155

CCC ACC TCA AGT TGC TCT GAT GGC ATG CCT GAA TGT AAC AGC CCT ATC TGG TCC AGA AAG  602
Pro Thr Ser Ser Cys Ser Asp Gly Met Pro Glu Cys Asn Ser Pro Ile Trp Ser Arg Lys
    160                 165                 170                 175
```

```
AGC AGC AGT TTT GAC AGC GTC TAC TGT CCT GAT GTA CCA AAT GTA TAT GCC ACG GAT AAA    662
Ser Ser Ser Phe Asp Ser Val Tyr Cys Pro Asp Val Pro Asn Val Tyr Ala Thr Asp Lys
    180                 185                 190                 195

AGC TCC TTA TCC AGC TTG GAT TGC TTA TCC AGC ATA GTG GAT CGG ATC ACC AAC TCA GAG    722
Ser Ser Leu Ser Ser Leu Asp Cys Leu Ser Ser Ile Val Asp Arg Ile Thr Asn Ser Glu
    200                 205                 210                 215

CAA CCT GGA TTG CCT CTC CAG GAT CCA GCC TCT CTC TCC CCA GTT GCC AGC ACC GAT TCT    782
Gln Pro Gly Leu Pro Leu Gln Asp Pro Ala Ser Leu Ser Pro Val Ala Ser Thr Asp Ser
    220                 225                 230                 235

CAA CCT GCA ACT CCA GGG GCC TCT AGT TCC AGG CTC ATC TAT CAT GTG CTA TGAACTAAAA     843
Gln Pro Ala Thr Pro Gly Ala Ser Ser Ser Arg Leu Ile Tyr His Val Leu
    240                 245                 250                 255

ATCTAGATCA GTTCTGCCAG AAGGCCTATT ACACAGGAGG AAGGAGGTAC CAAAAACCCC    903

AAAGCAAGAC AACCTGTAAA TAAACATTTC TTTTCTGTTG TAAATTTGTA AATAGTTATC    963

TTGCCACTTT ATAAGAAAGT GTATTTAAAA AGTCATTATT GCAATTTATA CTTTCTTTTT    1023

TTCTTCTTTT CCTTCATCTT TGCTTTAGAT ATATAGTTCC AATGATATTA TTTCTTATAG    1083

GGGCCGTTCA TCAAAGGTAG TTTGTTGCAA TGCTTAACTT ATATATTTTT ATAAATATTG    1143

CTTATCAAAA TATTACCTCT GATGTTTAGT GCTTTTTTTT CTTTAAAACA TTAGAATAGA    1203

TGTAAATCAG TTATAGGGAG TTTTAAATAT ATTTAACTGT CTTGCTTTTC TTTAATCTTT    1263

TGATTTATAT TGTGTTAAGT AAAATATAAC AATACTGCCT AATGGTATAT ATTTTAACAT    1323

TTCTTATAAG AAATACATTT TTAATCTAAG CACAAAATAG TACTTTGTGG ATGATTTCAA    1383

GATATAAGAG ATTTTTGGAA ACTCCACCAT AAATAAAATT GTTTAAGTGA GGAAATATTC    1443

TGGTTTTATG ATTTTGTTAA AAGAACCCCC TAATGGAATT GGCAGTCACT GATATGGTGT    1503
```

```
CTTTAAGGTG GGGCTGAAAG TACTGAAACA GTGGGTTTTG AAGATAAATC TGAATAGCAT   1563
CTCGCGGACT CAGTTTAGGT CCTGTTGGTG TCACAGATGA AAATACCACA TTTCTACTTC   1623
AAGCATATTT CAAAGGTGTC TGCTAACTAA AATTTTTCTT TTATCTTAAT GTTGTACCAT   1683
ATAACATAAA GTTGTGTGTA TGTGGAGCAA AAGTAGAAGG TATTCAGGAC AATAACAGAT   1743
TTTTAAACAA AAAGAATGTA CAATATTACA TCATTATAAT ATCTATAGTA CATTAGAAAT   1803
CATGAGTTAA GGTGATATAT ATCTATTTTC CCAAAAATAC CTTGATACAT TAGTATATTA   1863
ATATTGTCAA TAAAATATTT AAAGGTAAAA AAAAAAAAAA AAAAAAAAAA AAAAAAAAAA   1923
AAAAAAAA                                                            1931
```

(ix) Information for SEQ ID NO: 2

(i) Sequence characteristics:

(A) Length: 1931
(B) Type: nucleotide
(C) Standedness: single
(D) Topology: linear

(ii) Sequence description: SEQ ID NO: 2:

```
CCAGGCTCCG GTTTCTCCCC TATCTATCTC TCTGCTGTCC AGGCCCACCC CTTGCCTCTC     60
TGCAGACG ATG GAC ATG ATG GAC GGC TGC CAG TTC TCG CCC TCT GAG TAC TTC TAC GAC    119
GGC TCC TGC ATC CCA TCC CCC GAC GGT GAG TTC GGG GAC GAG TTT GAG CCG CGA GTG GCT    179
GCT TTC GGG GCT CAC AAG GCA GAC CTG CAA GGC TCA GAC GAG GAC GAG CAC GTG CGA GCA    239
CCC ACG GGC CAC CAC CAG GCC GGC CAC TGC CTC ATG TGG GCC TGC AAA GCA TGC AAA AGG    299
AAG TCC ACC ACC ATG GAT CGG CGG AAG GCG GCC ACC ATG CGC GAG CGG AGA CGC CTG AAG    359
AAG GTC AAC CAG GCT TTC GAC ACG CTC AAG CGG TGC ACC ACG ACC AAC CCT AAC CAG AGG    419
CTG CCC AAG GTG GAG ATC CTC AGG AAT GCC ATC CGC TAC ATT GAG AGT CTG CAG GAG CTG    479
CTT AGG GAA CAG GTG GAA AAC TAC TAT AGC CTG CCG GGG CAG AGC TGC TCT GAG CCC ACC    539
AGC CCC ACC TCA AGT TGC TCT GAT GGC ATG CCT GAA TGT AAC AGC CCT ATC TGG TCC AGA    599
AAG AGC AGC AGT TTT GAC AGC GTC TAC TGT CCT GAT GTA CCA AAT GTA TAT GCC ACG GAT    659
AAA AGC TCC TTA TCC AGC TTG GAT TGC TTA TCC AGC ATA GTG GAT CGG ATC ACC AAC TCA    719
GAG CAA CCT GGA TTG CCT CTC CAG GAT CCA GCC TCT CTC TCC CCA GTT GCC AGC ACC GAT    779
```

```
TCT CAA CCT GCA ACT CCA GGG GCC TCT AGT TCC AGG CTC ATC TAT CAT GTG CTA     833
TGAACTAAAA ATCTAGATCA GTTCTGCCAG AAGGCCTATT ACACAGGAGG AAGGAGGTAC           893
CAAAAACCCC AAAGCAAGAC AACCTGTAAA TAAACATTTC TTTTCTGTTG TAAATTTGTA           953
AATAGTTATC TTGCCACTTT ATAAGAAAGT GTATTTAAAA AGTCATTATT GCAATTTATA          1013
CTTTCTTTTT TTCTTCTTTT CCTTCATCTT TGCTTTAGAT ATATAGTTCC AATGATATTA          1073
TTTCTTATAG GGGCCGTTCA TCAAAGGTAG TTTGTTGCAA TGCTTAACTT ATATATTTTT          1133
ATAAATATTG CTTATCAAAA TATTACCTCT GATGTTTAGT GCTTTTTTTT CTTTAAAACA          1193
TTAGAATAGA TGTAAATCAG TTATAGGGAG TTTTAAATAT ATTTAACTGT CTTGCTTTTC          1253
TTTAATCTTT TGATTTATAT TGTGTTAAGT AAAATATAAC AATACTGCCT AATGGTATAT          1313
ATTTTAACAT TTCTTATAAG AAATACATTT TTAATCTAAG CACAAAATAG TACTTTGTGG          1373
ATGATTTCAA GATATAAGAG ATTTTTGGAA ACTCCACCAT AAATAAAATT GTTTAAGTGA          1433
GGAAATATTC TGGTTTTATG ATTTTGTTAA AAGAACCCCC TAATGGAATT GGCAGTCACT          1493
GATATGGTGT CTTTAAGGTG GGGCTGAAAG TACTGAAACA GTGGGTTTTG AAGATAAATC          1553
TGAATAGCAT CTCGCGGACT CAGTTTAGGT CCTGTTGGTG TCACAGATGA AAATACCACA          1613
TTTCTACTTC AAGCATATTT CAAAGGTGTC TGCTAACTAA AATTTTTCTT TTATCTTAAT          1673
GTTGTACCAT ATAACATAAA GTTGTGTGTA TGTGGAGCAA AAGTAGAAGG TATTCAGGAC          1733
AATAACAGAT TTTTAAACAA AAAGAATGTA CAATATTACA TCATTATAAT ATCTATAGTA          1793
CATTAGAAAT CATGAGTTAA GGTGATATAT ATCTATTTTC CCAAAAATAC CTTGATACAT          1853
TAGTATATTA ATATTGTCAA TAAAATATTT AAAGGTAAAA AAAAAAAAAA AAAAAAAAAA          1913
AAAAAAAAAA AAAAAAAA                                                        1931
```

(ix) Information for SEQ ID NO: 3

(i) Sequence characteristics:

(A) Length: 255
(B) Type: amino acid
(D) Topology: linear

(ii) Sequence description: SEQ ID NO: 3:

```
Met Asp Met Met Asp Gly Cys Gln Phe Ser Pro Ser Glu Tyr Phe Tyr
                 5                  10                  15

Asp Gly Ser Cys Ile Pro Ser Pro Asp Gly Glu Phe Gly Asp Glu Phe
            20                  25                  30

Glu Pro Arg Val Ala Ala Phe Gly Ala His Lys Ala Asp Leu Gln Gly
        35                  40                  45

Ser Asp Glu Asp Glu His Val Arg Ala Pro Thr Gly His His Gln Ala
    50                  55                  60

Gly His Cys Leu Met Trp Ala Cys Lys Ala Cys Lys Arg Lys Ser Thr
65                  70                  75                  80

Thr Met Asp Arg Arg Lys Ala Ala Thr Met Arg Glu Arg Arg Arg Leu
                85                  90                  95

Lys Lys Val Asn Gln Ala Phe Asp Thr Leu Lys Arg Cys Thr Thr Thr
            100                 105                 110

Asn Pro Asn Gln Arg Leu Pro Lys Val Glu Ile Leu Arg Asn Ala Ile
        115                 120                 125

Arg Tyr Ile Glu Ser Leu Gln Glu Leu Leu Arg Glu Gln Val Glu Asn
    130                 135                 140

Tyr Tyr Ser Leu Pro Gly Gln Ser Cys Ser Glu Pro Thr Ser Pro Thr
145                 150                 155                 160

Ser Ser Cys Ser Asp Gly Met Pro Glu Cys Asn Ser Pro Ile Trp Ser
                165                 170                 175

Arg Lys Ser Ser Ser Phe Asp Ser Val Tyr Cys Pro Asp Val Pro Asn
            180                 185                 190

Val Tyr Ala Thr Asp Lys Ser Ser Leu Ser Ser Leu Asp Cys Leu Ser
        195                 200                 205

Ser Ile Val Asp Arg Ile Thr Asn Ser Glu Gln Pro Gly Leu Pro Leu
    210                 215                 220

Gln Asp Pro Ala Ser Leu Ser Pro Val Ala Ser Thr Asp Ser Gln Pro
225                 230                 235                 240

Ala Thr Pro Gly Ala Ser Ser Ser Arg Leu Ile Tyr His Val Leu
                245                 250                 255
```

(ix) Information for SEQ ID NO: 4

(i) Sequence characteristics:

(A) Length: 385
(B) Type: nucleotide
(C) Strandedness: double
(D) Topology: linear

(ii) Sequence description: SEQ ID NO: 4:

```
TTGAGATTGG TAGAGAAGTC TTGGGTTGCT ATAGTGACCT TCACCCTTAT  50
TAAGACATTT AAATTCAAAG CTAGAATTTA CCCATCTGTG CTTTTCTCTT 100
TAGTAATATT TTGTCCCCTT TATTCATCTC AAAACTTTGC TCCTACAACA 150
CCTTATCTTG GAAAAAAATA TTTTAAAAAA TTTTAGTTCA AACTTTGAAT 200
ATCCATCTTC CAAATCTAAT GCATTTGTAA ATTAGCTATA ACATGGCATT 250
TCCTTAAAGA GCCAAGCTAT CAAGGCATTT CTCCAGCAGA CTCCTCCAGC 300
CACTCCCTTA GAGTCAGCAG TGTACAAAGA TGACCTCCTC CTAGAGGTTA 350
CTTCGAACGC ATTTTGCAGC TAGTCAGAGT CAGCT                 385
```

(ix) Information for SEQ ID NO: 5

(i) Sequence characteristics:

(A) Length: 814
(B) Type: nucleotide
(C) Strandedness: double
(D) Topology: linear

(ii) Sequence description: SEQ ID NO: 5:

```
GAGCTCAAGC CCTCCAGAAG CGATGCCTAG TTTCCAATTC TGAGTGACCT   50
GGGGCAAATG ACTTACTCCC CGAGCTTGGC TACCTTCTTT TAATAATGGG  100
TCCAGTTACA GTTGCCACAG AATTGTTGGA AGTCATAACT GAGTGCATAC  150
ATAGCAAGCG CTGGGAACAG AGCTTCCCAA GAGAAAACTC TTCTAATTGT  200
TTGCTTACAA ATATCCATGC TTAACAACTT TGTGCTTTGT GGAAAGAAAT  250
CGCCTAAAGA CAAGCCAGAA GCATAACTTC TATCTTCCAC TTAAACTTGA  300
AGGAGCAAAT TTATTTGTGC CCGAATCTTC AATTTGAACA CAGGTCTGAT  350
TTGCTTTGAC CAGTGTGTGA CAGTCTGGAC TACAGACCCA AAGAAAGTGC  400
TACTATAGCT GCTGACCAGA ACCTGTGGTC CCTGGCTGAG AATTTCTGGA  450
TGAGAATATC TACCCACTAC TGTGCTTCTG AGGTGGCAAC GTGCACACTT  500
TGGAATGTTT CCACTCCCCT GGATGGGTCC TTTAGGAACT CAATTCTTTT  550
ATCTGGTGTA GCCTGGGTAC CTATTCATTT CAAATGCATC TACTTGTTAA  600
CAGTTCTAAA AATAAGGTGC AGATGAATTC CCACTGCCCA ATTAAGCTGT  650
CCATTGGTAT GAAAAGGAGC CAGATTTTCA AGGGATGGAT GGTGATGGTG  700
GTGGTGGAGG TAGTGTCTTT GTCTTGGAGA TGACAGAGCT TGGGAAAGGG  750
GACAGCGGCA GGCACGTTTG TAGCAAACTT TGGTGACTGA CATTCTCCTA  800
CCCCTGATTT TGCT                                         814
```

(ix) Information for SEQ ID NO: 6

(i) Sequence characteristics:

(A) Length: 946
(B) Type: nucleotide
(C) Strandedness: double
(D) Topology: linear

(ii) Sequence description: SEQ ID NO: 6:

```
GGGAAAAGGG AAGCAAGCCC TGGAAGAGTT CATCATCTAA ACCTGGTTTC   50
TTAGAATACA AATCTTAGAG ACGCAGTCAG GGTGGATCTT CCGCCTGCTC  100
AGGTCCATCA CAGGAGGAGG CACTCAGCAA ACAGGCACAG AAGCGGGAAG  150
GAGAGGAAGC CAGGTGTGTT CCTGGTCACC TGGCCCTCCT GTGCCCACAC  200
CCTTCAGATG AGCAGAGAGA AAACTCAGAG CAAGCTGTTT TTGGAGAAAA  250
GGAACCTGCC AGGTTACCCC CAATAACATA CAGCCTACAT AATTGCAATC  300
TGATGAGAAA CAAAACAGAT TAAAAAAAAA AAATCTAGAG CCACTTCATT  350
ATTGAAAGTG CCACTTGAAG TTTTTAGCAA CTGTTATTTG GCCTAATGGC  400
TGAAAAAATT CCTGACACAA CTAAAGTTCA CGAAAAAGAG AAATACCTGA  450
AAATAAAGTT GGTCCCTCAT GTCTCATATT GGGAAGACGG GTCCGCTTTT  500
GGTGTCAACT GAAATGGGTA GCTGTATTTA CCGATTTTCT TTTTAAATGC  550
GGAGAGCAAT TGTACAAGTT GGATTTCCTT CGTTGCTTTT GTCTGTGACA  600
CCCTCTAAGT GTCCCTTGGT TTAGGTAATC TAAAGAGTGC AAGTAAGCGG  650
ATTGGGGAGC CATCACCTCC GCCCCAGCAG AAACGCAGAA ACCATTAGCG  700
TCAAAAGGGG CAGTAAACAG CGACTGTCTC TAGAGAAAAG GCGGAGGTTT  750
GCCCAGACAG CACTGGCGGG GGTTGCGGTG GGATATGCTA ATAGTGCGGG  800
GCCACAGGGG CGGCCTCCCT CCCCCAAGAA TATATAAAGA GCCCCAGCCC  850
CAGCGCGCAG ACCCCAGGCC GCCACGTCTG CCCTTGTTAA TTACCAGAGA  900
CACCGACCAG GGAGCTCGGC CCGGGATTTC CCTGCCCTCA GCGGCG      946
```

(ix) Information for SEQ ID NO: 7

(i) Sequence characteristics:

(A) Length: 569
(B) Type: nucleotide
(C) Strandedness: double
(D) Topology: linear

(ii) Sequence description: SEQ ID NO: 7:

```
CCAGGCTCCG GTTTCTCCCC TATCTATCTC TCTGCTGTCC AGGCCCACCC            50
CTTGCCTCTC TGCAGACGAT GGACATG ATG GAC GGC TGC CAG TTC TCG CCC    101
TCT GAG TAC TTC TAC GAC GGC TCC TGC ATC CCA TCC CCC GAC GGT GAG  149
TTC GGG GAC GAG TTT GAG CCG CGA GTG GCT GCT TTC GGG GCT CAC AAG  197
GCA GAC CTG CAA GGC TCA GAC GAG GAC GAG CAC GTG CGA GCA CCC ACG  245
GGC CAC CAC CAG GCC GGC CAC TGC CTC ATG TGG GCC TGC AAA GCA TGC  293
AAA AGG AAG TCC ACC ACC ATG GAT CGG CGG AAG GCG GCC ACC ATG CGC  341
GAG CGG AGA CGC CTG AAG AAG GTC AAC CAG GCT TTC GAC ACG CTC AAG  389
CGG TGC ACC ACG ACC AAC CCT AAC CAG AGG CTG CCC AAG GTG GAG ATC  437
CTC AGG AAT GCC ATC CGC TAC ATT GAG AGT CTG CAG GAG CTG CTT AGG  485
GAA CAG GTG GAA AAC TAC TAT AGC CTG CCG GGG CAG AGC TGC TCT GAG  533
CCC ACC AGC CCC ACC TCA AGT TGC TCT GAT GGC ATG                  569
```

(ix) Information for SEQ ID NO: 8

(i) Sequence characteristics:

(A) Length: 361
(B) Type: nucleotide
(C) Strandedness: double
(D) Topology: linear

(ii) Sequence description: SEQ ID NO: 8:

```
GTAAGAGATG GCTCTGTACC TGCTAGGACC TTCCAACTTT TACAAAAATC   50
CTTACATCTC ATTTAGACCA GGTGTAGCAA CCAGATATTC AGTGGTTACT  100
GGTGGATAGT TGGCTATTGG GGGGCGGGTG GGGCTCACCA CATCTAGATG  150
AGAAGAGAGA TGCCACATTT AGACAGATGC CAGCACACAG ACAGCTGTTG  200
AACAGGATTT TGTCCAGACT TCCTACCTAC TCTAGGTGCA CACTGAAGGA  250
GCAGGTTGTC ACTTGAGATA GCTGGCTGTG AATGATCAGT CAGATTTCTC  300
CATTACTCAG TTATTCAGTG TTATACTACC AGTGGACCTT GACATACCTA  350
ACATCTTAAG G                                            361
```

(ix) Information for SEQ ID NO: 9

(i) Sequence characteristics:

(A) Length: 258
(B) Type: nucleotide
(C) Strandedness: double
(D) Topology: linear

(ii) Sequence description: SEQ ID NO: 9:

```
ATTTGCATGT AACCAGTGTA CACTGCTGCT GAATTCCACT CTCTTCTTTC   50
TGCTGCCTCT CTCCTCTTCT CCAAGCACAG AGATTGACCT CAGTGCCCTT  100
GCAATTGGTG AGGCTAGCCC TTCCTAAATC AAGGCAATAA AGGTGATTGA  150
GAGTGGTCAG TTTATGGAGC TGGTGGGCAA GCACTGCCTC ACCCGAGATT  200
GGAGCACCTT TTGCCAAGAC CTGAAAACAA ACGCCTTCTT CTGTGTCTTT  250
TATTATAG                                                258
```

(ix) Information for SEQ ID NO: 10

(i) Sequence characteristics:

(A) Length: 75
(B) Type: nucleotide
(C) Strandedness: double
(D) Topology: linear

(ii) Sequence description: SEQ ID NO: 10:

CCT GAA TGT AAC AGC CCT ATC TGG TCC AGA AAG AGC AGC AGT TTT GAC 48

AGC GTC TAC TGT CCT GAT GTA CCA AAT 75

(ix) Information for SEQ ID NO: 11

(i) Sequence characteristics:

(A) Length: 393
(B) Type: nucleotide
(C) Strandedness: double
(D) Topology: linear

(ii) Sequence description: SEQ ID NO: 11:

GGGTAAGACC AATGACTTCA AGAGAGTTAA GACCAGGTTC AACTTCAGAT 50

TAGCCTATCA AGTAGAGTCT GGTTCTTCCA TGCAGTACTG GGAAGAGACA 100

GATGGTATTA TGGGGTTTGG CAGGGCAAAA TAAACACACA TCTTCTACTC 150

AAATCCTCCT AACAGATACA TCCATGATAT ACACACAGAC GTGAGCTTTT 200

GCTTGAAATA TTACCCAGGT GCTTCTCCAC TCCCCAACTT CATCCCCATG 250

AATTGCTAGA TATTTGTTGC AAATTTCTAC CAGGCTTTCT GTGACCACCT 300

GACCTTTGGG TTTCAAAGGT GGTGACCTGC AGTTTAAGCG GCAGCATAAG 350

CAAGGATTTT TTTTTTTTTT AATGGTTTTC TCCTTGTGTC CTT 393

(ix) Information for SEQ ID NO: 12

(i) Sequence characteristics:

(A) Length: 1245
(B) Type: nucleotide
(C) Strandedness: double
(D) Topology: linear

(ii) Sequence description: SEQ ID NO: 12:

```
GTA TAT GCC ACG GAT AAA AGC TCC TTA TCC AGC TTG GAT TGC TTA TCC    48
AGC ATA GTG GAT CGG ATC ACC AAC TCA GAG CAA CCT GGA TTG CCT CTC    96
CAG GAT CCA GCC TCT CTC TCC CCA GTT GCC AGC ACC GAT TCT CAA CCT   144
GCA ACT CCA GGG GCC TCT AGT TCC AGG CTC ATC TAT CAT GTG CTA TGA   192
ACTAAAAATC TAGATCAGTT CTGCCAGAAG GCCTATTACA CAGGAGGAAG GAGGTACCAA 252
AAACCCCAAA GCAAGACAAC CTGTAAATAA ACATTTCTTT TCTGTTGTAA ATTTGTAAAT 312
AGTTATCTTG CCACTTTATA AGAAAGTGTA TTTAAAAAGT CATTATTGCA ATTTATACTT 372
TCTTTTTTTC TTCTTTTCCT TCATCTTTGC TTTAGATATA TAGTTCCAAT GATATTATTT 432
CTTATAGGGG CCGTTCATCA AAGGTAGTTT GTTGCAATGC TTAACTTATA TATTTTTATA 492
AATATTGCTT ATCAAAATAT TACCTCTGAT GTTTAGTGCT TTTTTTTCTT TAAAACATTA 552
GAATAGATGT AAATCAGTTA TAGGGAGTTT TAAATATATT TAACTGTCTT GCTTTTCTTT 612
AATCTTTTGA TTTATATTGT GTTAAGTAAA ATATAACAAT ACTGCCTAAT GGTATATATT 672
TTAACATTTC TTATAAGAAA TACATTTTTA ATCTAAGCAC AAAATAGTAC TTTGTGGATG 732
ATTTCAAGAT ATAAGAGATT TTTGGAAACT CCACCATAAA TAAAATTGTT TAAGTGAGGA 792
AATATTCTGG TTTTATGATT TTGTTAAAAG AACCCCCTAA TGGAATTGGC AGTCACTGAT 852
ATGGTGTCTT TAAGGTGGGG CTGAAAGTAC TGAAACAGTG GGTTTTGAAG ATAAATCTGA 912
ATAGCATCTC GCGGACTCAG TTTAGGTCCT GTTGGTGTCA CAGATGAAAA TACCACATTT 972
CTACTTCAAG CATATTTCAA AGGTGTCTGC TAACTAAAAT TTTTCTTTTA TCTTAATGTT 1032
GTACCATATA ACATAAAGTT GTGTGTATGT GGAGCAAAAG TAGAAGGTAT TCAGGACAAT 1092
AACAGATTTT TAAACAAAAA GAATGTACAA TATTACATCA TTATAATATC TATAGTACAT 1152
TAGAAATCAT GAGTTAAGGT GATATATATC TATTTTCCCA AAAATACCTT GATACATTAG 1212
TATATTAATA TTGTCAATAA AATATTTAAA GGT                              1245
```

## Claims

1. Cloned DNA comprising a nucleotide sequence encoding bmyf-active protein.

2. The DNA of claim 1 wherein the sequence encodes bmyf protein.

3. The DNA of claim 1 wherein the cloned DNA is cDNA.

4. The DNA of claim 1 wherein the cloned DNA is cDNA having the nucleotide sequence encoding bmyf protein shown in SEQ ID NO 1.

5. The DNA of claim 1 wherein the cloned DNA is genomic DNA.

6. The DNA of claim 5 wherein the cloned DNA comprises one or more of the sequences selected from the group of SEQ ID NOS 3-11.

7. A cultured cell transformed with the cloned DNA of claim 1.

8. DNA comprising the promoter of the bmyf gene.

9. The DNA of claim 8 wherein the promoter comprises one or more sequences selected from the group of sequences in SEQ ID NOS. 3, 4 and 5.

10. The DNA of claim 8 wherein the promoter comprises a bmyf-active promoter.

11. The DNA of claim 8 further comprising a coding region under control of said promoter.

12. The DNA of claim 11 wherein the coding region is a heterologous coding region.

13. The DNA of claim 11 wherein the coding region encodes a reporter gene.

14. The DNA of claim 11 wherein the coding region encodes bmyf-active protein.

15. The DNA of claim 11 wherein the coding region encodes bmyf protein.

16. A cultured cell transformed with the promoter and coding region of claim 11.

17. A purified protein having an amino acid sequence comprising the amino acid sequence of bmyf-active protein.

18. The protein of claim 17 wherein the amino acid sequence comprises the amino acid sequence of bmyf protein.

19. A proteinaceous composition comprising a polyclonal or monoclonal antibody specific for bmyf protein.

20. The antibody of claim 19 comprising a monoclonal antibody.

21. The antibody of claim 19 comprising a polyclonal antibody directed to a peptide comprising the bmyf amino acids 1-19 of SEQ ID NO. 3.

22. The antibody of claim 19 comprising a polyclonal antibody directed to a peptide comprising the bmyf amino acids 101-118 of SEQ ID NO. 3.

23. The antibody of claim 19 comprising a polyclonal antibody directed to a peptide comprising the bmyf amino acids 154-169 of SEQ ID NO. 3.

Restriction map of bmyf gene and flanking sequences in recombinant clone λDIIBmyf1

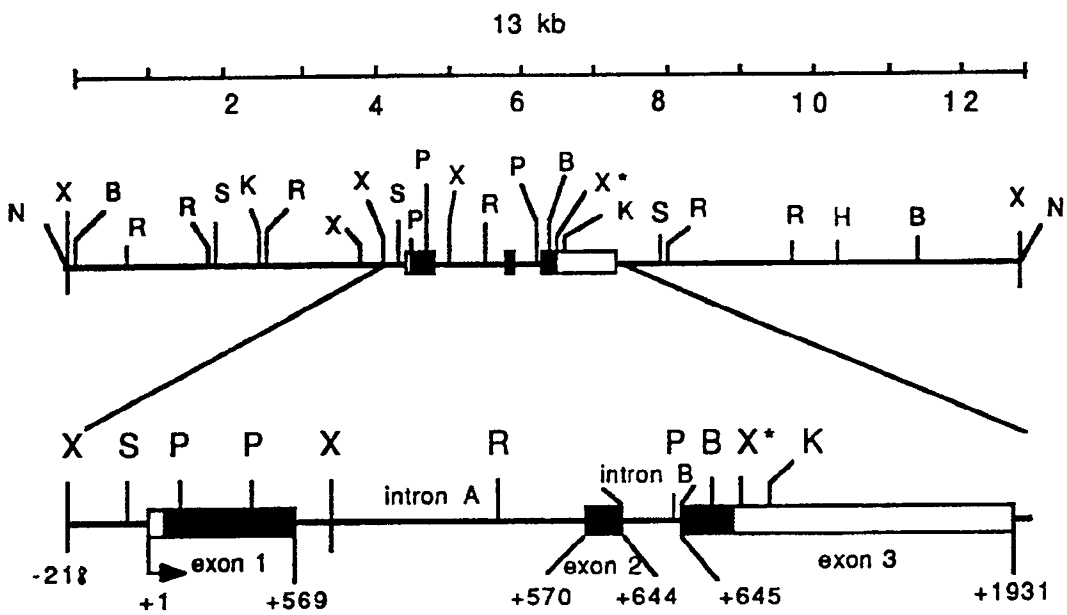


Positive (+) numbers identify nucleotide positions in bmyf cDNA sequence; minus (-) numbers identify nucleotide positions upstream from +1 of bmyf cDNA.

Restriction enzyme sites: B, BamHI
H, HindIII
K, KpnI
N, NotI
P, PstI
R, EcoRI
S, SacI
X, XbaI

■ amino acid coding

□ 5' or 3' untranslated

intron A = ~1,000 bp
intron B = ~390 bp

FIGURE 1

European Patent Office

# EUROPEAN SEARCH REPORT

Application Number

EP 90 31 4166

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| P,X | NUCLEIC ACIDS RESEARCH. vol. 18, no. 11, 1990, ARLINGTON, VIRGINIA pages 3147 - 3153; CLARK, T.G. et al.: "A BOVINE HOMOLOG TO THE HUMAN MYOGENIC DETERMINATION FACTOR MYF-5 : SEQUENCE CONSERVATION AND 3' PROCESSING OF TRANSCRIPTS" * the whole document * | 1-7, 17-23 | C12N15/63 C07K13/00 C12N15/86 C12N5/10 C12N1/21 C12N15/12 C12P21/08 A61K39/00 |
| P,X | BR39:106876 CLARK, T.G. et al. :"Isolation of a bovine homolog to the human myogenic factor myf-5." Symposium on signal transduction and gene ac tivation in development; symp. mol. cell. biol. STEAMBOAT SPRINGS, USA,March 31-april 7 1990. J CELL BIOCHEM SUPPL 0 14 PART E | 1-7, 17-23 | |
| X | EMBO JOURNAL. vol. 8, no. 3, March 1989, EYNSHAM, OXFORD GB pages 701 - 709; BRAUN, T. et al.: "A novel human muscle factor related to but distinct from MyoD1 induces myogenic conversion in 10T1/2 fibroblasts" * the whole document * | 1, 3, 5, 7, 17 | |
| | | | TECHNICAL FIELDS SEARCHED (Int. Cl.5 ) |
| | | | C07K |

The present search report has been drawn up for all claims

| Place of search | Date of completion of the search | Examiner |
|---|---|---|
| THE HAGUE | 13 FEBRUARY 1991 | CHAMBONNET F.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)